# EUROPEAN PATENT APPLICATION

(11) **EP 4 173 707 A1**
(43) Date of publication of application: **03.05.2023**
(21) Application number: 21204997.7
(22) Date of filing: 27.10.2021
(51) Int. Cl.: B01L 3/00, A61B 10/00, G01N 1/02

(54) **DEVICE FOR COLLECTING BIOLOGICAL SAMPLES**

(71) Applicant: BIC Violex Single Member S.A., 14569 Anoixi (GR)
(72) Inventor: Katsikas, Georgios, 145 69 Anoixi (GR); Saltas, Efthymios, 145 69 Anoixi (GR); Papageorgis Papadopoulos, Phaedon, 145 69 Anoixi (GR)
(74) Representative: Peterreins Schley

(57) **Abstract**

The present disclosure relates to a device for collecting one or more biological samples comprising a housing and a collector element, wherein the collector element is designed to collect one or more biological samples. The device comprises a container for storing liquid and a channel for guiding the liquid towards the collector element.

## Description

### Background of the present disclosure

The present disclosure relates to device for collecting one or more biological samples, which may be subsequently used for molecular biology processing techniques such as nucleic acid amplification and/or detection.

Polymerase Chain Reaction (PCR) is a known method of amplifying nucleic acids, employed to test for the presence of specific nucleic acids (DNA or RNA) in a biological sample. It is used among other purposes as a diagnostic method to identify markers for pathogens or diseases. Other methods of amplifying nucleic acid samples include isothermal amplification methods such as Recombinase Polymerase Amplification (RPA) and Loop-Mediated Isothermal Amplification (LAMP).

Devices for collecting one or more biological samples - in particular for collecting saliva from the mouth of the consumer - are known from WO 2020/191232 A1, WO 2020/106345 A1, WO 2020/238035 A1 and EP 3 028 030 A1. Those devices comprise a wick as collector element. However, the wick is very soft and is difficult to maintain and fasten into the housing of the device.

The object of the present disclosure is to provide a device for collecting one or more biological samples, wherein the one or more biological samples can be tested and/or analyzed more easily.

### Summary of the present disclosure

The present disclosure is directed to a device for collecting one or more biological samples as defined in independent claim 1. The dependent claims depict s embodiments of the present disclosure.

According to the present disclosure, a device for collecting one or more biological samples is provided comprising a housing and a collector element, wherein the collector element is designed to collect one or more biological samples. The device comprises a container for storing liquid and a channel for guiding the liquid to the collector element.

As will be explained below in detail, the collector element may be designed to allow the passage of the liquid so that at least part of the liquid can pass through the channel and through the collector element to wash out the one or more biological samples out of the collector element at least partially for testing/analyzing purposes. For testing/analyzing the one or more biological samples, a reagent may be contained in a cap which can be mounted on the housing for covering the collector element. When the mixture of liquid from the container and of the at least one biological sample reaches the internal volume of the cap, the reagent is released to the mixture for testing/analyzing the one or more samples on site. In other examples, the one or more samples may also be tested/analyzed in a laboratory.

It should be noted that the housing may consist of several separate housing parts. The collector element may generally have an elongated shape extending from the first end to the second end of the collector element. The cross-sectional shape of the collector element (in a plane which is perpendicular to the axis of the collector element) may at least partially be circular, oval or rectangular (in examples, with rounded corners). The section of the collector element at its first end may have a conical section. In addition or alternatively, the first end of the collector element may be pointed.

The purpose of the collector element is to collect and/or absorb one or more biological samples. In case of dry biological samples, the biological samples may stick on the outer surface of the first end of the collector element. In case of biological samples which are contained in a liquid, the biological samples may be absorbed within the collector element. Therefore, the collector element should allow the passage of liquid at least partially in order to take up absorb a greater amount of the biological sample(s).

Suitable materials for the collector element include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. Most specifically, the collector element may be made of a combination of PE and PP, wherein the PP may be the core portion and the PE may be the outer coating, due to its particular properties on absorption of liquid via its capillary structure, and adsorption which is minimal with regards to nucleic acids. The collector element may comprise fibers (in examples, pressed fibers) made of one or more of these materials. The material of the collector element may be porous or may have an internal channel structure, and may have hydrophilic properties in order to absorb a large amount of liquid biological samples more quickly. The porosity may be at least 50%, more specifically at least 70%, most specifically at least 80%. In examples, the density of the collector element may be between 0.1 to 0.25 g/cm³.

The collector element may extend between a first end and a second end, the first end of the collector element protruding out of the housing for collecting the one or more biological samples, the second end of the collector element being arranged within the housing. The collector element may allow the passage of the liquid from the container, in particular a buffer solution and/or a reagent solution, through the collector element in order to drain the biological sample(s) out of the collector element. In particular, the collector element may allow the passage of liquid from its second end which is arranged in the housing, to its first end which protrudes out of the housing, or from the outer surface near the second end to the first end.

The container may initially be in a closed state for storing the liquid and may further comprise a release mechanism for opening the container or a passage from the container to the channel so that liquid stored in the container can enter the channel from the container and reach the collector element. The liquid can thus be released when needed, i.e. after the one or more samples have been collected, and when a user wants to analyze/test the sample(s).

In particular, the container may have an opening which is closed by a membrane, wherein the release mechanism comprises a cutting or penetrating member for opening the container by a cutting or penetrating actuation. This can - for example - be achieved by moving the container from a first position into (or further into) the housing to a second position, wherein the cutting/penetrating member may be fixedly arranged within the housing. In this case, the membrane is closed in the first position of the container, and wherein the membrane is cut open or penetrated by the cutting or penetrating member in the second position of the container.

The housing and the container may also have a mechanism to retain the container in its first position such that the initial actuation force required to move the container into the housing is higher than the subsequent actuation force needed to cut open or penetrate the membrane. This feature ensures that the first position is maintained as a preset position. As an example, the retaining mechanism may comprise two internal protrusions on the inner side of the housing forming a circumferential groove between the protrusions, and a circumferential protrusion on the outer side of the container, wherein the circumferential protrusion is held within the circumferential groove of the housing. Therefore, when moving the container from its first position to its second position, the protrusion on side of the container needs to be pushed or moved over the circumferential protrusion on the housing which is closer to the cutting/penetrating member. This actuation requires an increased initial force which also avoids an undesired release of the liquid from the container.

In addition or alternatively, the housing may comprise a locking mechanism for maintaining the container in its first position, and which can be unlocked so that the container can be moved into its second position. This locking mechanism requires an unlocking actuation by a user which is different compared to a pushing or moving actuation on the container itself. For example, the locking mechanism may comprise at least one lever which can be operated by a user to disengage a protrusion on the container (which protrusion may be identical to the previously mentioned protrusion on the container). As another example, the locking mechanism may comprise a bayonet connection such that the container must be rotated first to unlock the axial position of the container so that the container can subsequently be moved in axial direction into the housing.

The container may be slidably arranged within the housing. Alternatively, the container and the housing may be connected over a threading so the container must be rotated with respect to the housing for moving the container into the housing.

The liquid stored in the container can reach the collector element through the channel when the release mechanism is open. At least part of the liquid may pass through the collector element by gravity acting on the liquid when the container is in a position vertically above the collector element. Alternatively or in addition, the container may be flexible such that the volume of the container can be reduced to squeeze liquid from the container into the channel. In particular, the volume of the container may be reduced such that liquid is released from the container into the channel and passes through the collector element. This alternative may assist to force either more liquid or a required amount of liquid more quickly through the collector element.

The device may further comprise a cap element which can be fitted on the housing on the side on which the collector element is arranged. The cap element may comprise a reagent or an element comprising a reagent which is released to the mixture of liquid from the container and of the at least one biological sample. An element for indicating and/or measuring a reaction between a reagent and the at least one biological sample may also be arranged within or on the cap element, in particular an element indicating the pH value of the mixture of the liquid from the container and of the at least one biological sample. With these features, the device can be used not only for collecting biological samples, but also to analyze/test the samples on site (i.e. without the need of sending the device to a laboratory).

The device of the present disclosure may be in a state in which the container does not yet contain the liquid. Alternatively, the container may already comprise a liquid, in particular a buffer solution and/or a reagent solution, i.e. the device may be in prefilled state.

According to this disclosure, various amplification methods may be used (i.e. the device of the present disclosure may be designed for carrying out one of these methods), for example a recombinase polymerase amplification (RPA) which is a low temperature DNA and/or RNA amplification technique, or a ligase chain reaction (LCR).

The buffer liquid stored in the container may contain purified water, milli-q water and buffer (e.g. TRIS-C1/TE buffer), for example a Tris-HCl buffer. When LCR method is applied, buffer also contains PEG as crowding agent.

The reagents located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Possible materials for the collector element have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide means of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

### Short description of the figures

- Figure 1: shows a device for collecting one or more biological samples according to an embodiment of the present disclosure;
- Figure 2: shows a cross-section through a device for collecting one or more biological samples according to an embodiment of the present disclosure;
- Figure 3: shows a retaining mechanism for retaining the container in the first position according to an embodiment of the present disclosure;
- Figure 4: shows a container according to a first embodiment of the present disclosure;
- Figure 5: shows a container according to a second embodiment of the present disclosure;
- Figures 6 and 7: show a locking mechanism for a container according to the second embodiment of the present disclosure;
- Figure 8: shows a container according to a third embodiment of the present disclosure;
- Figure 9: shows a container according to a fourth embodiment of the present disclosure which is connected to the housing over a bayonet connection;
- Figure 10: shows a cap element according to an embodiment the present disclosure including the parts which may be arranged within the cap element;
- Figures 11a to 11d and Figures 12a to 12c: show the parts which may be are arranged within the cap element according to an embodiment of the present disclosure.

### Detailed description of the present disclosure

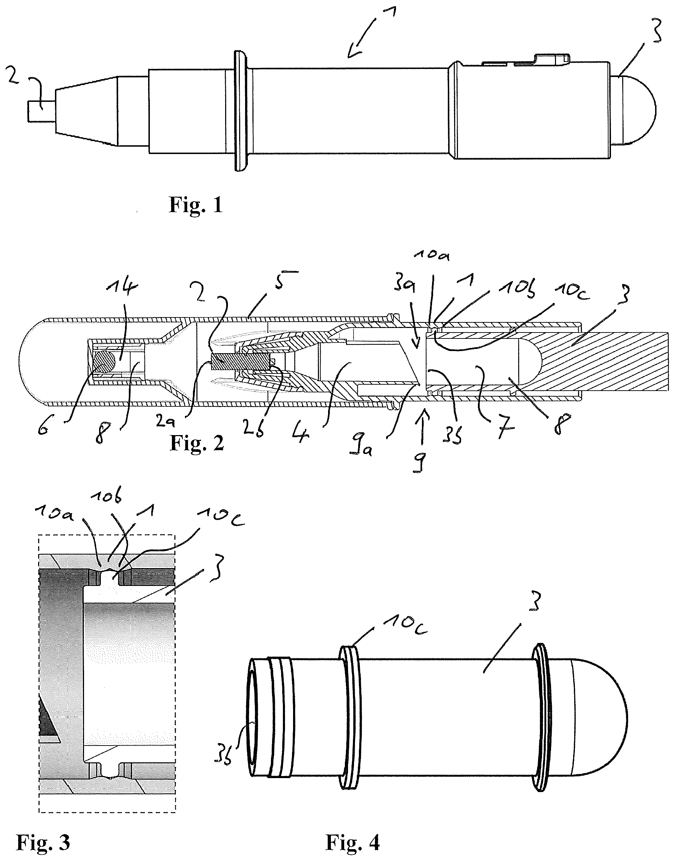

Figures 1 shows a device for collecting one or more biological samples according to an embodiment of the present disclosure. Figure 2 shows a cross-section through a device for collecting one or more biological samples according to another embodiment of the present disclosure which is a variation of the embodiment of Figure 1.

The device of Figures 1 and 2 comprises a housing 1 and a collector element 2, wherein the collector element is designed to collect one or more biological samples. The device comprises a container 3 for storing liquid 7 and a channel 4 for guiding the liquid to the collector element.

The collector element 2 may be designed to allow the passage of the liquid 7 so that at least part of the liquid can pass through the channel 4 and through the collector element 2 to wash out the one or more biological samples out of the collector element 2 at least partially for testing/analyzing purposes. For testing/analyzing the one or more biological samples, a reagent 6 may be contained in a cap 5 which can be mounted on the housing 1 for covering the collector element 2. When the mixture of liquid 7 from the container and of the at least one biological sample reaches the internal volume 8 of the cap 5, the reagent 6 is released to the mixture for testing/analyzing the one or more samples on site. Alternatively, the one or more samples may also be tested/analyzed in a laboratory.

It should be noted that the housing 1 may consist of several separate housing parts. In particular, the part of the housing holding the collector element 2 may consist of several separate parts for securely mounting the collector element 2. The collector element 2 may generally have an elongated shape extending from the first end 2a to the second end 2b of the collector element. The cross-sectional shape of the collector element (in a plane which is perpendicular to the axis of the collector element) may at least partially be circular, oval or rectangular (in examples, with rounded corners). The section of the collector element at its first end may have a conical section. In addition or alternatively, the first end of the collector element may be pointed.

The purpose of the collector element 2 is to collect and/or absorb one or more biological samples. In case of dry biological samples, the biological samples may stick on the outer surface of the first end of the collector element. In case of biological samples which are contained in a liquid, the biological samples may be absorbed within the collector element. Therefore, the collector element should allow the passage of liquid at least partially in order to take up absorb a greater amount of the biological sample(s). The material of the collector element may be porous or may have an internal channel structure, and may have hydrophilic properties in order to absorb a large amount of liquid biological samples more quickly.

Suitable materials for the collector element 2 include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. Most specifically, the collector element is made of a combination of PE and PP, wherein the PP is the core portion and the PE is the outer coating, due to its particular properties on absorption of liquid via its capillary structure, and adsorption which is minimal with regards to nucleic acids. The collector element may comprise fibers (in examples, pressed fibers) made of one or more of these materials. The material of the collector element may be porous or may have an internal channel structure, and may have hydrophilic properties in order to absorb a large amount of liquid biological samples more quickly. The porosity should be at least 50%, more specifically at least 70%, most specifically at least 80%.

The collector element 2 may have two ends, the first end 2a of the collector element protruding out of the housing for collecting the one or more biological samples, the second end 2b of the collector element being arranged within the housing. The collector element 2 may allow the passage of the liquid 7 from the container 3, in particular a buffer solution and/or a reagent solution, through the collector element 2 in order to drain the biological sample(s) out of the collector element. In particular, the collector element may allow the passage of liquid from its second end 2b which is arranged in the housing, to its first end 2a which protrudes out of the housing, or from the outer surface near the second end to the first end.

In the embodiments shown in Figures 1 and 2, the container 3 may initially be in a closed state for storing the liquid and may further comprise a release mechanism 9 for opening the container 3 or a passage from the container to the channel 4 so that liquid stored in the container 3 can enter the channel 4 from the container and reach the collector element 2. The liquid 7 can thus be released when needed, i.e. after the one or more samples have been collected, and when a user wants to analyze/test the sample(s).

In particular, the container 3 may have an opening 3a which is closed by a membrane 3b which is sealed to the rim of the opening 3a, wherein the release mechanism 9 comprises a cutting or penetrating member 9a for opening the container by a cutting or penetrating actuation. This can - for example - be achieved by moving the container 3 from a first position (as shown in Figure 2) into (or further into) the housing 1 to a second position (i.e. towards the left side from it position shown in Figure 2), wherein the cutting/penetrating member 9a may be fixedly arranged within the housing. In this case, the membrane 3b is closed in the first position of the container, wherein the membrane 3b is cut open or penetrated by the cutting or penetrating member 9a in the second position of the container 3.

As shown in Figures 3 and 4, in all embodiments of the present disclosure, the housing 1 and the container 3 may also have a mechanism 10 to retain the container in its first position such that the initial actuation force required to move the container into the housing is higher than the subsequent actuation force needed to cut open or penetrate the membrane. This feature ensures that the first position is maintained as a preset position. As an example, the retaining mechanism may comprise two internal protrusions 10a, 10b on the inner side of the housing forming a circumferential groove between the protrusions, and a circumferential protrusion 10c on the outer side of the container, wherein the circumferential protrusion is held within the circumferential groove of the housing. Therefore, when moving the container from its first position to its second position, the protrusion 10c on side of the container needs to be pushed or moved over the circumferential protrusion 10a on the housing which is closer to the cutting/penetrating member. This actuation requires an increased initial force which also avoids an undesired release of the liquid from the container. The protrusions 10a, 10b and 10c may extend continuously in circumferential direction. In other examples, one or more of these protrusions may be discontinuous.

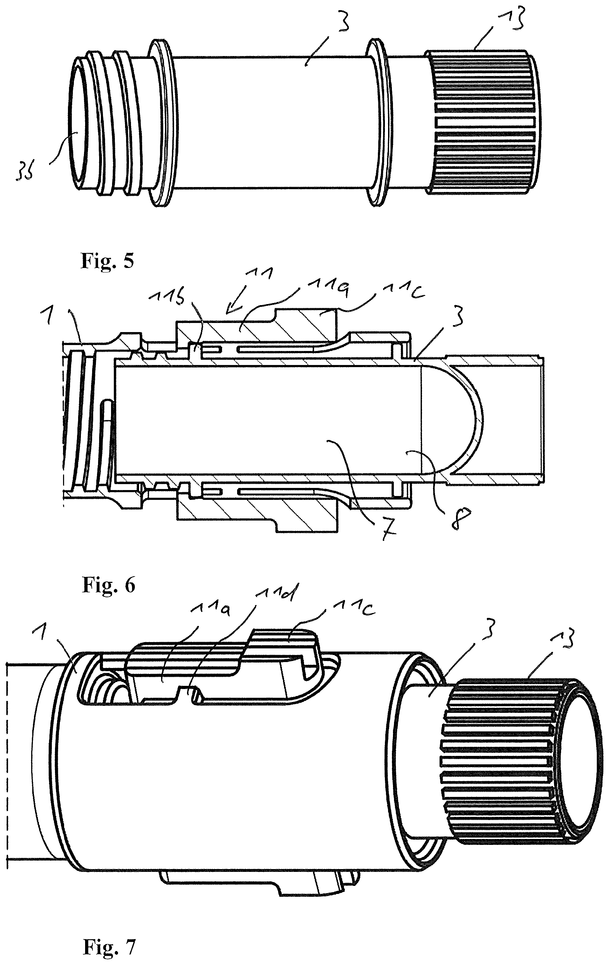

The container may be slidably arranged within the housing (as shown in Figures 2 to 4). In other examples, as shown in Figures 5 and 6, the container and the housing may be connected over a threading so the container must be rotated with respect to the housing for moving the container into the housing.

In addition or alternatively, as shown in Figures 6 and 7, the housing may comprise a locking mechanism 11 for maintaining the container in its first position, and which can be unlocked so that the container 3 can be moved into its second position. This locking mechanism 11 requires an unlocking actuation by a user which is different compared to a pushing or moving actuation on the container itself. For example, the locking mechanism 11 may comprise at least one lever 11a which can be operated by a user to disengage a protrusion 11b on the container (which protrusion may be identical to the previously mentioned protrusion on the container). In the shown embodiment, lever 11a may have an elevated part 11c for being operated by a user. Lever 11a is connected to the housing by linkages 11 d on both sides acting as a hinge such that the lever 11a rotates slightly when being operated by a user to disengage the protrusion 11b.

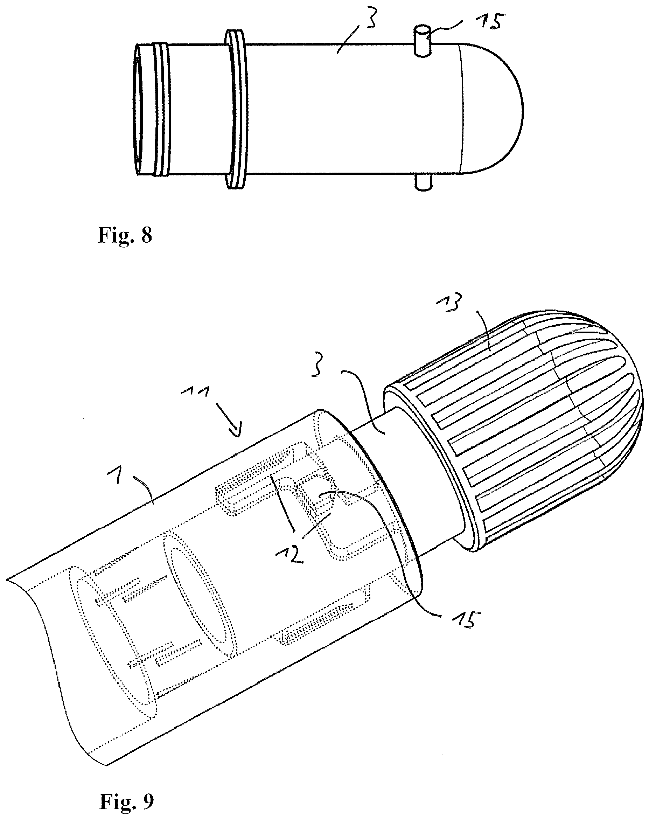

In addition or alternatively, as shown in Figures 8 and 9, the locking mechanism 11 may comprise a bayonet connection such that the container 3 must be rotated first to unlock the axial position of the container so that the container can subsequently be moved in axial direction into the housing. In this embodiment, the housing may have one or more internal grooves 12 generally having the shape of an "L" or of a "J" which engages one or more protrusions 15 of the container.

In all emodiments of the present disclosure, the container may have a plurality of ribs 13 extending in axial direction to facilitate the rotation of the container (see for example Figures 5, 7 and 9).

The liquid 7 stored in the container 3 can reach the collector element through the channel 4 when the release mechanism 9 has opened the fluid passage to the channel 4. At least part of the liquid may pass through the collector element 2 by gravity acting on the liquid 7 when the container is in a position vertically above the collector element 2. Alternatively or in addition, the container may be flexible such that the internal volume 8 of the container can be reduced to squeeze liquid from the container into the channel. In particular, the volume of the container may be reduced such that liquid is released from the container into the channel and passes through the collector element 2. This alternative may assist to force either more liquid or a required amount of liquid more quickly through the collector element.

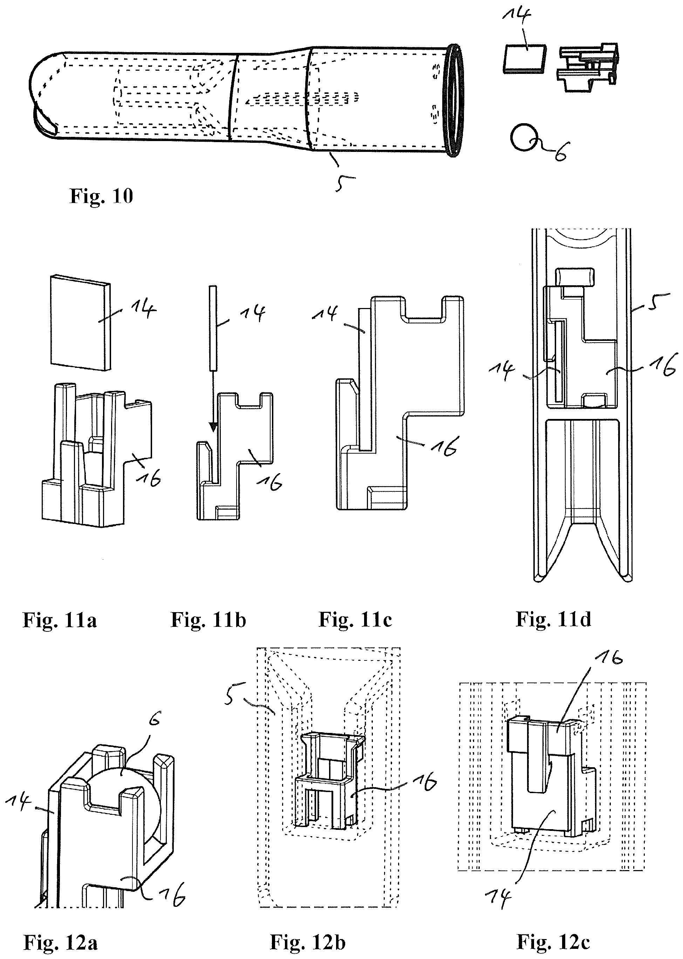

As shown in Figures 2 and 10, the device may further comprise a cap element 5 which can be fitted on the housing 1 on the side on which the collector element 2 is arranged. An element 14 for indicating and/or measuring a reaction between a reagent and the at least one biological sample may be arranged within or on the cap element 5, in particular an element 14 indicating the pH value of the mixture of the liquid from the container and of the at least one biological sample. In addition or alternatively, the cap element 5 may comprises a reagent 6 or an element comprising a reagent which is released to the mixture of liquid from the container and of the at least one biological sample, and the cap element 5 may comprise a window which is arranged such that a user can at least partially see the element for indicating and/or measuring a reaction between the reagent and the at least one biological sample. With these features, the device can be used not only for collecting biological samples, but also to analyze/test the samples on site (i.e. without the need of sending the device to a laboratory).

The device of the present disclosure may be in a state in which the container does not yet contain the liquid. In other examples, the container may already comprise the liquid 7, in particular a buffer solution and/or a reagent solution, i.e. the device may be in prefilled state.

According to this disclosure, various amplification methods may be used (i.e. the device of the present disclosure may be designed for carrying out one of these methods), for example a recombinase polymerase amplification (RPA) which is a low temperature DNA and/or RNA amplification technique, or a ligase chain reaction (LCR).

The liquid stored in the container may be a buffer liquid and may contain purified water, milli-q water and buffer (e.g. TRIS-C1 / TE buffer), for example a Tris-HCl buffer. When LCR method is applied, buffer also contains PEG as crowding agent.

The reagent or reagents 6 located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Figures 11a to 11d and Figures 12a to 12c show the parts which may be are arranged within the cap element 5 according to an embodiment of the present disclosure. The element 14 for indicating and/or measuring a reaction and the reagent(s) 6 may be held by a holder 16 on which those elements/parts can be premounted before this subunit is mounted within the cap element 5. The reagent(s) may have the shape of a sphere which is held by the holder 16, in the shown example by elongated sides which hold the reagent in place, e.g. by a snap fit, press fit and/or shape fit feature. The element 14 may also be held by the holder 16 in place, for example by a rib which exerts a certain pressing force on element 14. The holder 16 may be mounted in a reaction chamber of the cap element 5, and - for example - be held therein by a snap fit, press fit and/or shape fit feature (see esp. Figure 12c).

Possible materials for the collector element 2 have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide means of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

Although the device of the present disclosure is defined in the following claims, it should be understood that the present disclosure can alternatively and/or in addition be defined according to the following embodiments:
1. A device for collecting one or more biological samples comprising a housing and a collector element,
   wherein the collector element is designed to collect one or more biological samples,
   characterized in that the device comprises a container for storing liquid and a channel for guiding the liquid to the collector element.
2. Device according to embodiment 1, wherein the collector element has two ends, the first end of the collector element protruding out of the housing for collecting the one or more biological samples, the second end of the collector element being arranged within the housing.
3. Device according to one of the preceding embodiments, wherein the collector element is designed to allow the passage of the liquid so that at least part of the liquid can pass through the channel and through the collector element to wash out the one or more biological samples out of the collector element at least partially for testing/analysis purposes.
4. Device according to one of the preceding embodiments, wherein the container is initially in a closed state for storing the liquid and further comprises a release mechanism for opening the container or a passage from the container to the channel so that liquid stored in the container can enter the channel from the container and reach the collector element.
5. Device according to one of the preceding embodiments, wherein the container has an opening which is closed by a membrane, and wherein the release mechanism comprises a cutting or penetrating member for opening the container by a cutting or penetrating actuation.
6. Device according to embodiment 5, wherein the container and the housing are designed such that the container can be moved with respect to the housing from a first closed position in which the membrane is closed to a second open position in which the membrane is open.
7. Device according to embodiment 5 or 6, further comprising a retention mechanism provided between the container and the housing and configured to retain the container within the housing in the first closed position, wherein the retention mechanism is further configured such that when the container is moved towards the second open position, the release mechanism cuts or penetrates the membrane.
8. Device according to one of the preceding embodiments, wherein the housing and the container has a mechanism to retain the container in its first position such that the initial actuation force required to move the container into the housing is higher than the subsequent actuation force needed to cut open or penetrate the membrane.
9. Device according to one of the preceding embodiments, wherein the retaining mechanism comprises two internal protrusions on the inner side of the housing forming a circumferential groove, and a circumferential protrusion on the outer side of the container, wherein the circumferential protrusion is held within the circumferential groove of the housing in the first closed position.
10. Device according to one of the preceding embodiments, further comprising a locking mechanism for securing the container in the first closed position, and which is configured to unlock the retention mechanism so that the container can be moved into the second open position.
11. Device according to embodiment 10, wherein the locking mechanism comprises at least one lever configured to be operated by a user to disengage the circumferential protrusion of the container from the circumferential groove of the housing.
12. Device according to one of the preceding embodiments, wherein the locking mechanism comprises one or more pins with a corresponding slot to define one or more bayonet connections such that to unlock the retention mechanism, the container is rotated to unlock the axial position and is subsequently moved in axial direction into the housing.
13. Device according to one of the preceding embodiments, wherein the release mechanism comprises matching threads respectively arranged on an inner side of the housing and an outer side of the container, the matching threads configured to penetrate the membrane when the container is rotated with respect to the housing.
14. Device according to one of the preceding embodiments, wherein the container and the housing are connected over a threading so the container must be rotated with respect to the housing for moving the container into the housing.
15. Device according to one of the preceding embodiments, wherein liquid stored in the container can reach the collector element through the channel when the container is in the second open position, wherein at least part of the liquid can pass through the collector element by gravity acting on the liquid when the device is in a vertical position with the container being arranged above the collector element.
16. Device according to one of the preceding embodiments, wherein the volume of the container can be reduced to squeeze liquid from the container into the channel.
17. Device according to one of the preceding embodiments, wherein the volume of the container can be reduced such that liquid is released from the container into the channel and passes through the collector element.
18. Device according to one of the preceding embodiments, further comprising a cap element which can be fitted on the housing on the side on which the collector element is arranged.
19. Device according to one of the preceding embodiments, characterized in that an element for indicating and/or measuring a reaction between a reagent and the at least one biological sample is arranged within or on the cap element, in particular an element indicating the pH value of the mixture of the liquid from the container and of the at least one biological sample.
20. Device according to one of the preceding embodiments, characterized in that the cap element comprises a reagent or an element comprising a reagent which is released to the mixture of liquid from the container and of the at least one biological sample.
21. Device according to one of the preceding embodiments, wherein the cap element comprises a window which is arranged such that a user can at least partially see the element for indicating and/or measuring a reaction between the reagent and the at least one biological sample.
22. Device according to one of the preceding embodiments, further comprising a liquid stored in the container, in particular a buffer solution and/or a reagent solution.

## Claims

1. A device for collecting one or more biological samples comprising a housing and a collector element,
wherein the collector element is designed to collect one or more biological samples,
**characterized in that** the device comprises a container for storing liquid and a channel for guiding the liquid towards the collector element.

2. Device according to claim 1, wherein the collector element extends between a first end and a second end, the first end of the collector element protruding out of the housing for collecting the one or more biological samples, the second end of the collector element being arranged within the housing in fluid communication with the channel.

3. Device according to one of the preceding claims, wherein the collector element is designed to allow the passage of the liquid so that in use, at least part of the liquid can pass through the channel and through the collector element to wash the one or more biological samples out of the collector element at least partially.

4. Device according to one of the preceding claims, wherein the container is initially in a closed state for storing the liquid and further comprises a release mechanism for opening the container or a passage from the container to the channel so that liquid stored in the container can enter the channel and reach the collector element.

5. Device according to one of the preceding claims, wherein the container has an opening which is initially closed by a membrane, and wherein the release mechanism comprises a cutting or penetrating member for opening the container.

6. Device according to one of the preceding claims, wherein the container and the housing are designed such that the container can be moved with respect to the housing from a first closed position into the housing to a second open position in which the membrane is open.

7. Device according to one of the preceding claims, wherein the housing and the container has a mechanism to retain the container in its first position such that the initial actuation force required to move the container into the housing is higher than the subsequent actuation force needed to cut open or penetrate the membrane.

8. Device according to one of the preceding claims, further comprising a retention mechanism provided between the container and the housing and configured to retain the container within the housing in the first closed position, wherein the retention mechanism is further configured such that when the container is moved towards the second open position, the release mechanism cuts or penetrates the membrane.

9. Device according to one of the preceding claims, wherein the retaining mechanism comprises two internal protrusions on the inner side of the housing forming a circumferential groove, and a circumferential protrusion on the outer side of the container, wherein the circumferential protrusion is held within the circumferential groove of the housing in the first closed position.

10. Device according to one of the preceding claims, further comprising a locking mechanism for securing the container in the first closed position, and which is configured to unlock the retention mechanism so that the container can be moved into the second open position.

11. Device according to claim 10, wherein the locking mechanism comprises at least one lever which can be operated by a user to disengage a protrusion on the container.

12. Device according to one of the preceding claims, wherein the locking mechanism comprises a bayonet connection such that the container must be rotated first to unlock the axial position of the container so that the container can subsequently be moved in axial direction into the housing.

13. Device according to one of the preceding claims, wherein the container and the housing are connected over a threading so the container must be rotated with respect to the housing for moving the container into the housing.

14. Device according to one of the preceding claims, wherein liquid stored in the container can reach the collector element through the channel when the release mechanism is open, wherein at least part of the liquid can pass through the collector element by gravity acting on the liquid when the container is in a position vertically above the collector element.

15. Device according to one of the preceding claims, further comprising a cap element which can be fitted on the housing on the side on which the collector element is arranged, wherein the cap element comprises a reagent or an element comprising a reagent which can be released to a mixture of liquid from the container and of the at least one biological sample, and wherein an element for indicating and/or measuring a reaction between a reagent and the at least one biological sample is arranged within or on the cap element, in particular an element indicating the pH value of the mixture of the liquid from the container and of the at least one biological sample.
